# EUROPEAN PATENT APPLICATION

(11) **EP 4 826 428 A1**
(43) Date of publication of application: **21.10.2026**
(21) Application number: 25217180.6
(22) Date of filing: 20.11.2025
(51) Int. Cl.: G02B 7/02, G03B 17/56, H04N 23/55, H04N 23/57, A61B 90/00, G03B 15/14, F16M 13/04

(54) **IMAGING DEVICE, IMAGING APPRATUS, AND SURGICAL FIELD IMAGING APPRATUS**

(30) Priority: 17.04.2025 CN 202520738043 U; 17.04.2025 CN 202520738067 U; 06.11.2025 CN 202521195088 U; 06.11.2025 CN 202521195091 U; 06.11.2025 CN 202521195082 U
(71) Applicant: Nizi Medical Technology (Shanghai) Co., Ltd, Shanghai 201800 (CN)
(72) Inventor: LIU, Chaoqi, Shanghai, 201800 (CN); Li, Yajie, Shanghai, 201800 (CN); Yang, Sen, Shanghai, 201800 (CN); Gan, Xiaoqiang, Shanghai, 201800 (CN)
(74) Representative: Metida

(57) **Abstract**

The present disclosure discloses an imaging device, an imaging apparatus, and a surgical field imaging apparatus. A protective cover of the imaging device can be removed from a camera hole, such that one end of a lens can be exposed through the camera hole. Then, the user can grasp or clamp the exposed end of the lens to remove the lens from an imaging unit, and then remove amounting chamber through the camera hole. After removing the lens, the user can install other lenses on the imaging unit through the camera hole, and finally cover the protective cover back to the camera hole to achieve lens replacement.

## Description

### TECHNICAL FIELD

The present disclosure relates to the field of imaging device technology, and more particularly, to an imaging device, an imaging apparatus, and a surgical field imaging apparatus.

### BACKGROUND

With the continuous development of technology, gimbals have been increasingly widely used in fields such as photography, videography, and security. The gimbal drives the rotation of an imaging device through a brushless motor. In related technologies, the camera device used in conjunction with the gimbal is usually a compact camera device, which is lighter in weight, simpler in structure, and easier to control and drive. In some application scenarios, the camera device needs to capture high-resolution video images to display details. However, a lens module of the compact camera device is permanently encapsulated within the housing, preventing users from replacing lenses with different focal lengths, apertures, or focusing capabilities according to their needs. This constraint significantly compromises the functional versatility and imaging performance of the device.

### SUMMARY

The main purpose of the present disclosure is to propose an imaging device aimed at solving the technical problem of how to improve the performance of compact imaging devices.

To achieve the above objectives, the imaging device provided in the present disclosure includes:
a housing having a mounting chamber and a camera hole being in communication with the mounting chamber;
a lens module mounted in the mounting chamber, wherein the lens module includes a lens and an imaging unit, one end of the lens is located at the camera hole to capture optical images through the camera hole, and the other end of the lens is detachably connected to the imaging unit, allowing the lens to be replaceable; and
a protective cover detachably covered on the camera hole and having a transparent area corresponding to the lens.

In the technical solution of the imaging device of the present disclosure, the protective cover can be removed from the camera hole, such that one end of the lens can be exposed through the camera hole. Then, the user can grasp or clamp the exposed end of the lens to remove the lens from the imaging unit, and then remove the mounting chamber through the camera hole. After removing the lens, the user can install other lenses on the imaging unit through the camera hole, and finally cover the protective cover back to the camera hole to achieve lens replacement. Thus, by configuring the lens to be detachable from the imaging unit and the protective cover to be detachable from the housing, the lens can be replaced, facilitating the users to replace lenses with different focal lengths, apertures, and focusing abilities according to actual needs, thereby improving the use effect of the imaging device.

### BRIEF DESCRIPTION OF THE DRAWINGS

To more clearly illustrate the technical solutions in the embodiments of the present disclosure or the prior art, the accompanying drawings required for the description of the embodiments or the prior art will be briefly introduced below. It is evident that the accompanying drawings described below are merely some embodiments of the present disclosure. For those skilled in the art, other drawings can be obtained based on the structures shown in these accompanying drawings without creative effort. In the drawings:

In order to provide a clearer explanation of the embodiments or technical solutions in the present disclosure or existing technology, a brief introduction will be given to the accompanying drawings required for the description of the embodiments or existing technology. It is obvious that the accompanying drawings described below are only some embodiments of the present disclosure. For those skilled in the art, other drawings can be obtained based on the structures shown in these drawings without creative labor.
FIG. 1 is a schematic view of an imaging device in accordance with an embodiment of the present disclosure;
FIG. 2 is a schematic sectional view of the imaging device in accordance with an embodiment of the present disclosure;
FIG. 3 is a schematic exploded view of the imaging device in accordance with an embodiment of the present disclosure;
FIG. 4 is an exploded sectional view of a lens module in accordance with an embodiment of the present disclosure;
FIG. 5 is an exploded sectional view of the imaging device in accordance with an embodiment of the present disclosure;
FIG. 6 is a schematic view of an imaging apparatus in accordance with an embodiment of the present disclosure;
FIG. 7 is a schematic view of the imaging apparatus in accordance with an embodiment of the present disclosure;
FIG. 8 is a schematic sectional view of the imaging apparatus in accordance with an embodiment of the present disclosure;
FIG. 9 is an exploded sectional view of the imaging apparatus in accordance with an embodiment of the present disclosure;
FIG. 10 is a schematic view of a portable and comfortable head-mounted bracket in accordance with an embodiment of the present disclosure;
FIG. 11 is a schematic view of a portable and comfortable head-mounted bracket in accordance with another embodiment of the present disclosure;
FIG. 12 is a partially-exploded sectional view of the portable and comfortable head-mounted bracket in accordance with an embodiment of the present disclosure;
FIG. 13 is a partially schematic view of the portable and comfortable head-mounted bracket of the present disclosure;
FIG. 14 is a schematic view of a head-mounted bracket in accordance with an embodiment of the present disclosure;
FIG. 15 is a partially-exploded view of the head-mounted bracket of the present disclosure;
FIG. 16 is an exploded sectional view of the head-mounted bracket of the present disclosure;
FIG. 17 is a partially sectional view of the head-mounted bracket of the present disclosure;
FIG. 18 is a schematic view of a portable and comfortable head-mounted bracket in accordance with an embodiment of the present disclosure;
FIG. 19 is a schematic view of a portable and comfortable head-mounted bracket in accordance with another embodiment of the present disclosure;
FIG. 20 is a schematic sectional view of a front bracket in accordance with an embodiment of the present disclosure;
FIG. 21 is a circuit module diagram of a camera control box circuit in accordance with an embodiment of the present disclosure;
FIG. 22 is a circuit module diagram of a camera control box circuit in accordance with another embodiment of the present disclosure;
FIG. 23 is a circuit module diagram of a camera control box circuit in accordance with another yet embodiment of the present disclosure; and
FIG. 24 is a circuit module diagram of a camera control box circuit in accordance with a further yet embodiment of the present disclosure.

The realization of the purpose, functional features, and advantages of the present disclosure will be further explained in conjunction with the embodiments and with reference to the accompanying drawings.

### DETAILED DESCRIPTION OF THE EMBODIMENTS

To make the objectives, technical solutions, and advantages of the embodiments of the present disclosure clearer, the technical solutions in the embodiments of the present disclosure will be described clearly and comprehensively below in conjunction with the accompanying drawings. Obviously, the described embodiments are only a part of the embodiments of the present disclosure, not all of them. Based on the embodiments of the present disclosure, all other embodiments obtained by those of ordinary skill in the art without creative effort fall within the scope of protection of the present disclosure.

It should be noted that all directional indications (such as up, down, left, right, front, and back) in the embodiments of the present disclosure are only used to explain the relative positional relationships and motion conditions between various components in a specific posture (as shown in the accompanying drawings). If the specific posture changes, the directional indications will also change accordingly.

In addition, the descriptions involving "first", "second", etc. in the present disclosure are only used for descriptive purposes and cannot be understood as indicating or implying their relative importance or implicitly specifying the number of technical features indicated. Therefore, features defined as "first" or "second" may explicitly or implicitly include at least one such feature. Furthermore, "and/or" throughout the text includes three options, taking A and/or B as an example, including the technical solution of A, the technical solution of B, and the technical solution where both A and B are satisfied simultaneously. Additionally, technical solutions between various embodiments can be combined with each other, but this must be based on what ordinary technical personnel in the field can achieve. When the combination of technical solutions is contradictory or unachievable, it should be considered that such combination of technical solutions does not exist and is not within the scope of protection claimed in the present disclosure.

The present disclosure provides an imaging device 10 aimed at solving the technical problem of how to improve the performance of compact imaging devices.

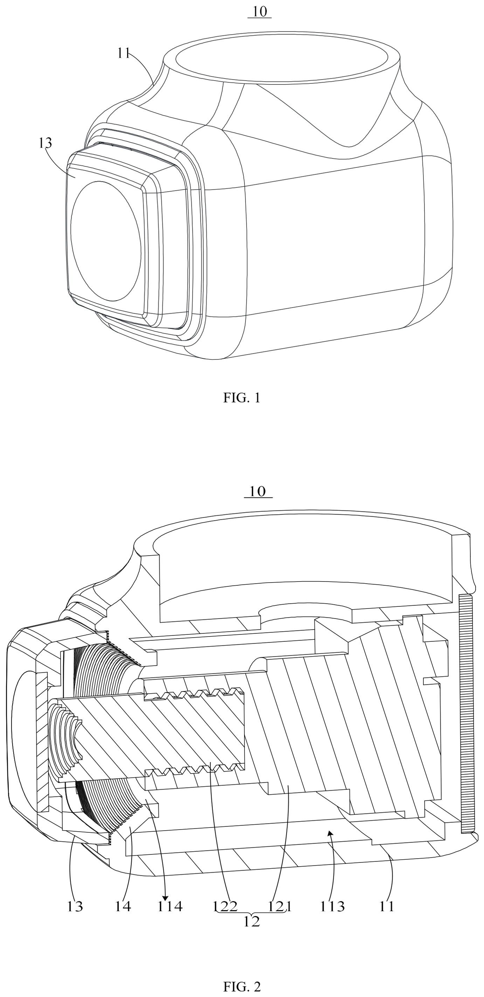

In the embodiments of the present disclosure, as shown in FIGS. 1 to 5, the imaging device 10 includes a housing 11, a lens module 12, and a protective cover 13. The housing 11 has a mounting chamber 113 and a camera hole 114 in communication with the mounting chamber 113. The lens module 12 is mounted in the mounting chamber 113, and includes a lens 122 and an imaging unit 121. One end of the lens 122 is located at the camera hole 114 to capture optical images through the camera hole 114, and the other end of the lens 122 is detachably connected to the imaging unit 121 to allow for replacement of the lens 122. The protective cover 13 is detachably covered on the camera hole 114 and has a transparent area corresponding to the lens 122.

In this embodiment, the housing 11 can form the overall appearance structure of the imaging device 10, and the lens module 12 can form images and record the images. The lens 122 is configured to capture optical images through the camera hole 114, and the imaging unit 121 is configured to record the optical images.

The protective cover 13 can provide dust prevention and protection for the lens 122. The transparent area of the protective cover 13 is a solid transparent area that allows light to pass there through. The transparent area can be formed through a transparent glass sheet.

The protective cover 13 can be removed from the camera hole 114, such that one end of the lens 122 can be exposed through the camera hole 114. Then, the user can grasp or clamp the exposed end of the lens 122 to remove the lens 122 from the imaging unit 121, and then remove the mounting chamber 113 through the camera hole 114. After removing the lens 122, the user can install other lenses 122 on the imaging unit 121 through the camera hole 114, and finally cover the protective cover 13 back to the camera hole 114 to replace the lens 122.Thus, the lens 122becomes replaceable by allowing the lens 122to be detachable from the imaging unit 121 and the protective cover 13 to be detachable from the housing 11. This facilitates users to interchange the lenses 122 with different focal lengths, apertures, and focusing capabilities according to the actual needs, thereby enhancing the operational effectiveness of the imaging device 10.

On the basis that the lens 122 and the imaging unit 121 can be detachably connected, the lens 122 can be connected to the imaging unit 121 through clamping connection or insertion connection, without limitation.

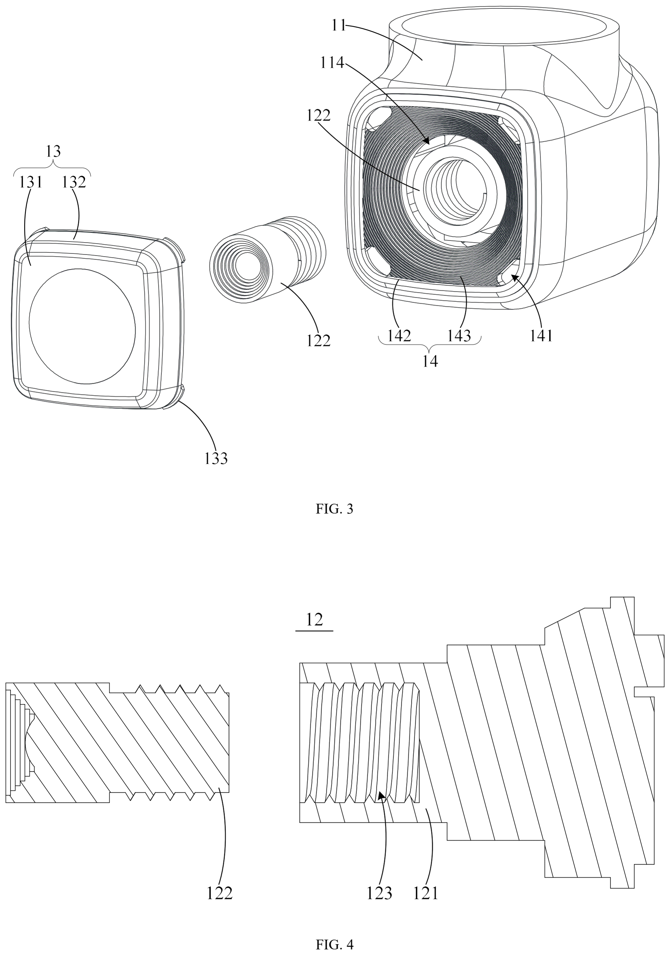

As shown in FIG. 4, a connection hole 123 is defined in the imaging unit 121, and one end of the lens 122 detachably engages with the connection hole 123.The combination of the lens 122 and the connection hole 123 can increase an engagement area between the lens 122 and the imaging unit 121, thereby improving the stability of the connection between the lens 122 and the imaging unit 121.

For example, an engagement depth between the lens 122 and the connection hole 123 can be adjusted, allowing for focus adjustment of the lens 122. An adjustable focusing gap is formed between one end of the lens 122 located at the camera hole 114 and the protective cover 13. By changing the engagement depth between the lens 122 and the connection hole 123, a distance between the lens 122 and the protective cover 13 can be adjusted, thereby adjusting a focal length of the lens 122.After removing the protective cover 13, users can manually adjust the focus of the lens 122, and then cover the protective cover 13 back on the camera hole 114, achieving the adjustable focus function of the imaging device 10, and further improving the use effect of the imaging device 10.

The engagement depth between the lens 122 and the connection hole 123 can be adjusted by directly applying a force to the lens 122 along a depth direction of the connection hole 123, or through other means.

A peripheral wall of the engagement portion between the lens 122 and the connection hole 123 is provided with external threads, a hole wall of the connection hole 123 is provided with internal threads, and the lens 122engages with the connection hole 123 through threaded engagement. In this way, the engagement depth between the lens 122 and the connection hole 123 can be adjusted by twisting the lens 122, thereby simplifying the focus adjustment process of the lens 122. In addition, the threaded engagement between the lens 122 and the connection hole 123 can also simplify the disassembly and assembly of the lens 122 and the connection hole 123, thereby facilitating the focusing and disassembly of the lens 122.

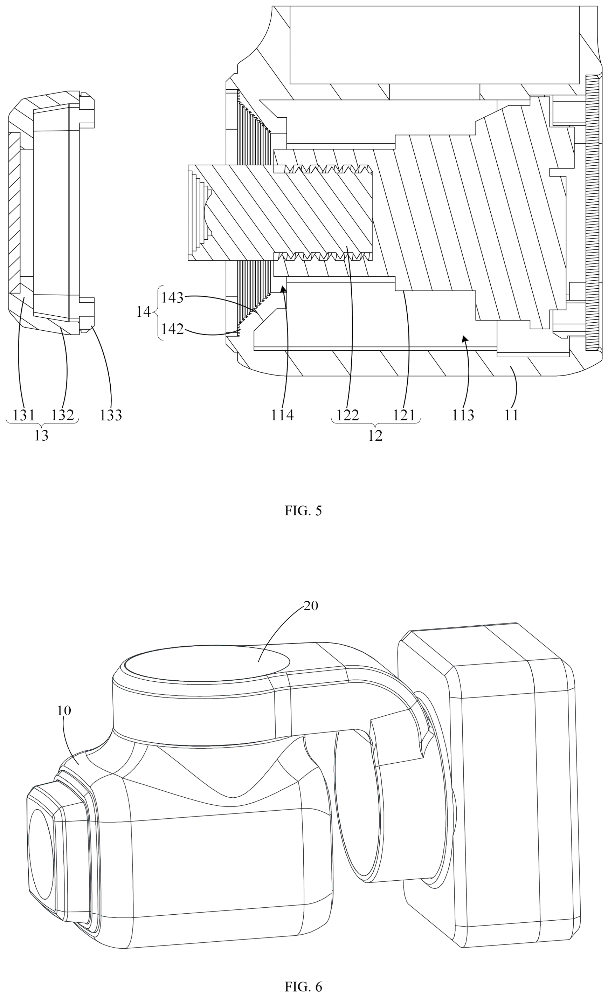

For example, as shown in FIGS. 3 and 5, the lens 122 extends out of the camera hole 114. The protective cover 13 includes a cover plate 131 and a surrounding plate 132. The surrounding plate 132 is connected to a periphery of the cover plate 131, and the cover plate 131 is covered on the transparent area. The cover plate 131 corresponds to the lens 122, and the surrounding plate 132 is arranged around the portion of the lens 122 extending out of the camera hole 114.

After removing the protective cover 13 from the camera hole 114, since the lens 122 extends out of the camera hole 114, the user can grasp the lens 122 from outside the camera hole 114 for focusing or disassembly conveniently, thereby facilitating the user's operation. A thickness of the surrounding plate 132 is greater than a length of the portion of the lens 122 extending out of the camera hole 114, thereby ensuring sufficient space between the cover plate 131 and the lens 122. Thus, a shape of the protective cover 13 can adapt to the portion of the lens 122 extending out of the camera hole 114, thereby ensuring the protective effect of the protective cover 13 on the lens 122.

As shown in FIG. 5, an aperture of the camera hole 114 gradually decreases from an end thereof away from the mounting chamber 113 towards an end thereof close to the mounting chamber 113. This allows the camera hole 114 to form an outward expanding shape, providing users with a certain operating space between the hole wall of the camera hole 114 and the peripheral wall of the lens 122, further facilitating the focus adjustment and disassembly of the lens 122.

For example, as shown in FIGS. 2 to 5, the imaging device 10 further includes an installation ring 14 arranged inside the camera hole 114 and surrounding the lens 122. An end of the surrounding plate 132 away from the cover plate 131 is detachably connected to the installation ring 14.During the disassembly and assembly process of the protective cover 13, a part of the housing 11 at the camera hole 114 also receives a force. The installation ring 14 can improve the structural strength of the housing 11 at the camera hole 114, prevent the part of the housing 11 at the camera hole 114 being easily damaged due to multiple times of disassembly and assembly of the protective cover 13, and improve the overall structural stability of the imaging device 10.

On the basis that the surrounding plate 132 and the installation ring 14 are detachably connected, the surrounding plate 132 and the installation ring 14 can be connected through clamping connection or threaded engagement, without limitation.

As shown in FIG. 3, a buckle 133 protrudes from the end of the surrounding plate 132 away from the cover plate 131. A buckle hole 141 adapted to the buckle 133 is defined in the installation ring 14, and the buckle 133 is detachably engaged in the buckle hole 141.In this way, the disassembly and assembly of the surrounding plate 132 and the installation ring 14 can be simplified to simplify the disassembly and assembly process of the protective cover 13.

In practical applications, as shown in FIG. 3, the surrounding plate 132 is shaped as a square, and the number of the buckles 133 is four. The four buckles 133 respectively protrude from four corners of the surrounding plate 132. The installation ring 14 includes an outer frame 142 and a ring body 143. The outer frame 142 is connected to the hole wall of the camera hole 114 at the end of the camera hole 114 away from the mounting chamber 113. One end of the ring body 143 is connected to the outer frame 142and the other end of the ring body 143 extends into the mounting chamber 113. The outer frame 142 is shaped as a square. The buckle holes 141 are defined in the outer frame 142. The number of the buckle holes 141 is four, and the four buckle holes 141 respectively correspond to the buckles 133 in position, and each buckle 133 is detachably connected to the corresponding buckle hole 141.Setting the number of buckles 133 and the buckle holes 141 to be both four can increase the clamping positions between the protective cover 13 and the installation ring 14, thereby improving the stability of the connection between the protective cover 13 and the mounting chamber 113. The four buckles 133 are arranged at the four corners of the square surrounding plate 132, which increases the structural strength of the positions where the four buckles 133 are located, allowing the surrounding plate 132 to be less prone to deformation and improving the structural stability of the protective cover 13.

As shown in FIG. 6, the present disclosure further provides an imaging apparatus, which includes an installation gimbal 20 and an imaging device 10. The specific structure of the imaging device 10 refers to the above embodiments. By adopting all the technical solutions of the above embodiments, the imaging apparatus at least has all the beneficial effects brought by the technical solutions of the above embodiments, which will not be repeated here. The installation gimbal 20 is fixedly mounted on an external structure, and the imaging device 10 can be rotatably mounted on the installation gimbal 20.

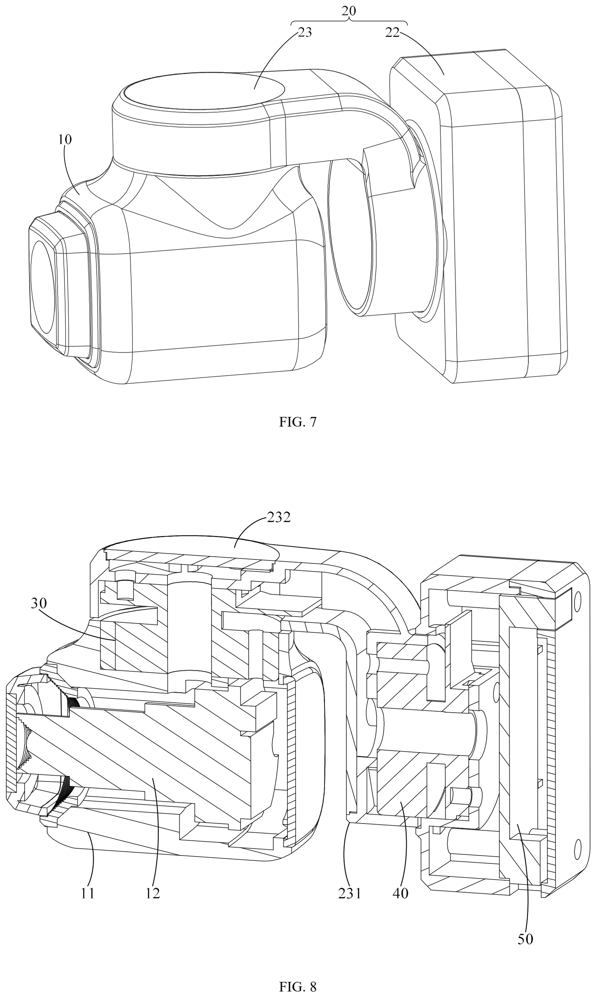

As shown in FIGS. 7 to 9, the present disclosure further provides an imaging apparatus aimed at solving the technical problem of achieving a compact imaging apparatus.

As shown in FIGS. 7 to 9, the imaging apparatus includes an imaging device 10, an installation gimbal 20, and a first brushless motor 30.

The imaging device 10 includes a housing 11 and a lens module 12 mounted inside the housing 11, and an outer surface of the housing 11 is recessed to form a first mounting groove 111.

The installation gimbal 20 is fixedly mounted to an external structure. A first through hole 21 is defined in the installation gimbal 20 and is in communication with an inner cavity of the installation gimbal 20. The first through hole 21 corresponds to an opening of the first mounting groove 111.

The first brushless motor 30 includes a first fixing seat 31, a first stator 32, and a first rotor 33. The first fixing seat 31 includes a first fixing plate 311 and a first connecting shaft 312 protruding from the first fixing plate 311. The first fixing plate 311 is mounted in the inner cavity of the installation gimbal 20, and the first connecting shaft 312 extends out of the installation gimbal 20 through the first through hole 21and extends into the first mounting groove 111. The first stator 32 and the first rotor 33 are received in the first mounting groove 111. The first stator 32 is fixed to the first connecting shaft 312, and the first rotor 33 is arranged around the first stator 32 and is rotatably connected to the first connecting shaft 312. The first rotor 33 is fixedly connected to the first mounting groove 111 to drive the imaging device 10 to rotate.

In this embodiment, the imaging device 10 can form images and record the images. The lens module 12 is mounted inside the housing 11 which can hide and protect the lens module 12.The installation gimbal 20 is configured to fix the imaging device 10 to the external structure, such as a drone or a head-mounted bracket. The imaging device 10 is mounted on the installation gimbal 20 through the first brushless motor 30, such that the imaging device 10 can rotate relative to the installation gimbal 20. Therefore, the user can adjust a shooting angle of the imaging device 10 by controlling the first brushless motor 30.

The first stator 32 of the first brushless motor 30 is fixed to the first connecting shaft 312, and the first rotor 33 is rotatably connected to the first connecting shaft 312 through a bearing, that is, the first rotor 33 can rotate relative to the first connecting shaft 312 and the first stator 32. Since the first rotor 33 is fixedly mounted in the first mounting groove 111,the imaging device 10 is driven to rotate synchronously as the first rotor 33 rotates. The first mounting groove 111 can hide and protect the first rotor 33 and the first stator 32.

The first stator 32 and the first rotor 33 are components with a relatively large volume in the first brushless motor 30. By receiving the first stator 32 and the first rotor 33 in the first mounting groove 111 of the imaging device 10, the first stator 32 and the first rotor 33 do not need to occupy the internal space of the installation gimbal 20, thereby reducing the overall volume of the installation gimbal 20 and achieving a compact design. In addition, the first brushless motor 30 can be prevented from being directly exposed, thus, the overall appearance structure of the imaging apparatus can be simpler and more compact. Compared to mounting the first stator 32 and the first rotor 33 in the installation gimbal 20, the volume of the imaging device 10 increases by a smaller proportion by mounting the first stator 32 and the first rotor 33 in the first mounting groove 111. In other words, the volume of the imaging device 10 does not significantly increase, which can keep the overall appearance of the imaging apparatus simple and compact, and facilitate the application and assembly of the imaging apparatus.

As shown in FIGS. 7 to 9, the installation gimbal 20 includes a fixing gimbal 22 and a movable gimbal 23. The fixing gimbal 22 is fixedly mounted to an external structure. The movable gimbal 23 is L-shaped and includes a connecting portion 231 and an installation portion 232 connected at an angle. The connecting portion 231 is rotatably mounted on the fixing gimbal 22. The imaging device 10 is located on a side of the connecting portion 231 away from the fixing gimbal 22. The first fixing plate 311 is mounted in an inner cavity of the installation portion 232, and the first through hole 21 is defined in the installation portion 232. A rotation axis of the connecting portion 231 is perpendicular to a rotation axis of the imaging device 10.

The imaging device 10 is located in an area formed by the connecting portion 231 and the installation portion 232. The movable gimbal 23 can rotate relative to the fixing gimbal 22, while the imaging device 10 can rotate relative to the movable gimbal 23. The rotation axis of the imaging device 10 is perpendicular to that of the movable gimbal 23, allowing for dual axis rotation control of the imaging device 10 and providing more selectable shooting angles.

In practical applications, as shown in FIGS. 7 to 9, one side of the connecting portion 231 facing the fixing gimbal 22 is recessed to form a second mounting groove 233. A second through hole 221 is defined in the fixing gimbal 22 and is in communication with an inner cavity of the fixing gimbal 22, and the second through hole 221 corresponds to an opening of the second mounting groove 233. The imaging apparatus further includes a second brushless motor 40, which includes a second fixing seat 41, a second stator 42, and a second rotor 43. The second fixing seat 41 includes a second fixing plate 411 and a second connecting shaft 412 protruding from the second fixing plate 411. The second fixing plate 411 is mounted in the inner cavity of the fixing gimbal 22, and the second connecting shaft 412 extends out of the fixing gimbal 22 through the second through hole 221 and extends into the second mounting groove 233. The second stator 42 and the second rotor 43 are received in the second mounting groove 233, the second stator 42 is fixed to the second connecting shaft 412, and the second rotor 43 is arranged around the second stator 42 and is rotatably connected to the second connecting shaft 412. The second rotor 43 is fixedly connected to the second mounting groove 233 to drive the movable gimbal 23 to rotate.

The second stator 42 of the second brushless motor 40 is fixed to the second connecting shaft 412, and the second rotor 43 is rotatably connected to the second connecting shaft 412 through a bearing, that is, the second rotor 43 can rotate relative to the second connecting shaft 412 and the second stator 42. Since the second rotor 43 is fixedly mounted in the second mounting groove 233, the imaging device 10 is driven to rotate synchronously as the second rotor 43 rotates. The second mounting groove 233 can hide and protect the second rotor 43 and the second stator 42.

The second stator 42 and the second rotor 43 are components with a relatively large volume in the second brushless motor 40. By receiving the second stator 42 and the second rotor 43 in the second mounting groove 233 of the connecting portion 231, the second stator 42 and the second rotor 43 do not need to occupy the internal space of the fixing gimbal 22, thereby reducing the overall volume of the gimbal 20 and achieving a compact design. In addition, the second brushless motor 40 can be prevented from being directly exposed, allowing the overall appearance structure of the imaging apparatus to be simpler and more compact.

For example, as shown in FIGS. 7 to 9, the imaging apparatus further includes a main control board 50 mounted inside the fixing gimbal 22. A second wiring channel 413 is formed in the second connecting shaft 412. One end of the second wiring channel 413runs through one end of the second connecting shaft 412, and the other end of the second wiring channel 413runs through the second fixing plate 411. A second wiring hole 234 is defined in a bottom of the second mounting groove 233, connecting the inner cavity of the connecting portion 231 with the second wiring channel 413. The first brushless motor 30 further includes a first electrical connector 34. One end of the first electrical connector 34 is mounted on the first fixing plate 311 and is electrically connected to the first stator 32. The other end of the first electrical connector 34 extends from inside the installation portion 232 along the connecting portion 231, the second wiring hole 234, and the second wiring channel 413 in sequence into the fixing gimbal 22, and is electrically connected to the main control board 50.

The main control board 50 provides electrical energy and transmits control signals to the first stator 32 through the first electrical connector 34. A Hall sensor is mounted on the first electrical connector 34 to detect a position of the first rotor 33. By obtaining the position of the first rotor 33, the main control board 50 can adjust a current delivered to the first stator 32 according to the position of the first rotor 33 to ensure the continuous rotation of the first rotor 33.

The first electrical connector 34 can be a flexible circuit board (FPC). A routing path of the first electrical connector 34 is from the installation portion 232 to the connecting portion 231, the second wiring hole 234, and the second wiring channel 413, and is finally connected to the main control board 50 inside the fixing gimbal 22.The second wiring hole 234 and the second wiring channel 413 pass through a rotation axis of the second rotor 43, such that the first electrical connector 34 can pass through the second wiring channel 413 without affecting the rotation of the second rotor 43, which can ensure the normal rotation of the second rotor 43 and the effective electrical connection between the first electrical connector 34 and the main control board 50.

As shown in FIGS. 7 to 9, a first wiring channel 313 is formed in the first connecting shaft 312. One end of the first wiring channel 313 runs through one end of the first connecting shaft 312, and the other end of the first wiring channel 313 runs through the first fixing plate 311. A first wiring hole 112 is defined in a bottom of the first mounting groove 111, connecting the inner cavity of the housing 11 with the first wiring channel 313. An electrical connection wire of the lens module 12 extends from inside the housing 11 through the first wiring hole 112, the first wiring channel 313, the installation portion 232, the connecting portion 231, and the second wiring hole 234 in sequence into the fixing gimbal 22 and is electrically connected to the main control board 50.

The main control board 50 is also configured to provide power to the lens module 12 and transmit control signals. A routing path of the electrical connection wire of the lens module 12 is from the housing 11 to the installation portion 232, the connecting portion 231, the second wiring hole 234, and the second wiring channel 413, and is finally electrically connected to the main control board 50 inside the fixing gimbal 22.The first wiring hole 112 and the first wiring channel 313 pass through the rotation axis of the first rotor 33, such that the electrical connection wire of the lens module 12 can pass through the first wiring channel 313 without affecting the rotation of the first rotor 33, which ensures the normal rotation of the first rotor 33 and the second rotor 43 and effective electrical connection between the electrical connection wire of the lens module 12 and the main control board 50.

In practical applications, as shown in FIGS. 7 to 9, the second brushless motor 40 further includes a second electrical connector 44. One end of the second electrical connector 44 is mounted on the second fixing plate 411 and is electrically connected to the second stator 42, and the other end of the second electrical connector 44 is electrically connected to the main control board 50.

The second electrical connector 44 is mounted inside the fixing gimbal 22. The main control board 50 provides electrical energy to the second stator 42 and transmits control signals through the second electrical connector 44. A Hall sensor is mounted on the second electrical connector 44 to detect a position of the second rotor 43. By obtaining the position of the second rotor 43, the main control board 50 can adjust the current delivered to the second stator 42 according to the position of the second rotor 43 to ensure the continuous rotation of the second rotor 43.

For example, as shown in FIGS. 7 to 9, a first protrusion 235 is arranged inside the installation portion 232, and the first fixing plate 311 is fixedly connected to the first protrusion 235.The first protrusion 235 can enhance the structural strength of the connection portion between the movable gimbal 23 and the first fixing seat 31, thereby improving the mounting stability of the first fixing seat 31 inside the movable gimbal 23.

As shown in FIGS. 7 to 9, a second protrusion 222 is arranged inside the fixing gimbal 22, and the second fixing plate 411 is fixedly connected to the second protrusion 222.The second protrusion 222 can enhance the structural strength of the connection portion between the fixing gimbal 22 and the second fixed seat 41, thereby improving the mounting stability of the second fixed seat 41 inside the fixing gimbal 22.

In practical applications, the lens module 12 includes an imaging unit 121 and a lens 122 detachably mounted on the imaging unit 121. A camera hole is defined in the housing 11, and one end of the lens 122 away from the imaging unit 121 is located at the camera hole. The imaging device 10 also includes a protective cover 13 detachably covered on the camera hole. The protective cover 13 has a transparent area corresponding to the lens 122.

By opening the protective cover 13, the lens 122 can be exposed to the camera hole and then be removed from the imaging unit 121 to allow for replacement of lenses 122 of different specifications. This facilitates users to replace the corresponding lens 122 according to their own needs, thereby improving the performance of the imaging device 10.

The present disclosure further provides a surgical field imaging apparatus including a head-mounted bracket and an imaging device. The specific structure of the imaging device refers to the above embodiments. By adopting all the technical solutions of the above embodiments, the surgical field imaging apparatus has at least all the beneficial effects brought by the technical solutions of the above embodiments, which will not be repeated here. The head-mounted bracket is configured to be worn on the human head, and the installation gimbal 20 of the imaging apparatus is fixedly mounted on a front side wall of the head-mounted bracket. The installation gimbal 20 has a stable anti-shake function. When the user's head rotates or shakes and drives the imaging apparatus to move, the sensors inside the installation gimbal 20 detect the change in the movement state of the installation gimbal 20 and generate a detection signal to send to the main control board. After receiving the detection signal, the main control board controls the corresponding brushless motor to move in the opposite direction to offset the shaking caused by the installation gimbal 20. Therefore, the imaging device can be stabilized at the target position and angle to ensure that the final captured image is clear and smooth.

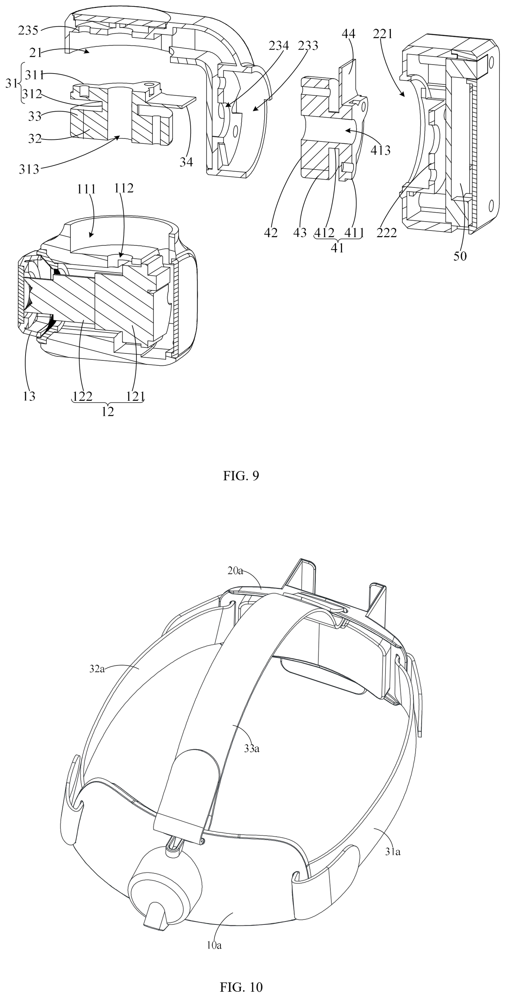

As shown in FIGS. 10 to 13, the present disclosure provides a portable and comfortable head-mounted bracket, aiming to solve the technical problem of how to improve the wearing comfort of the head-mounted bracket.

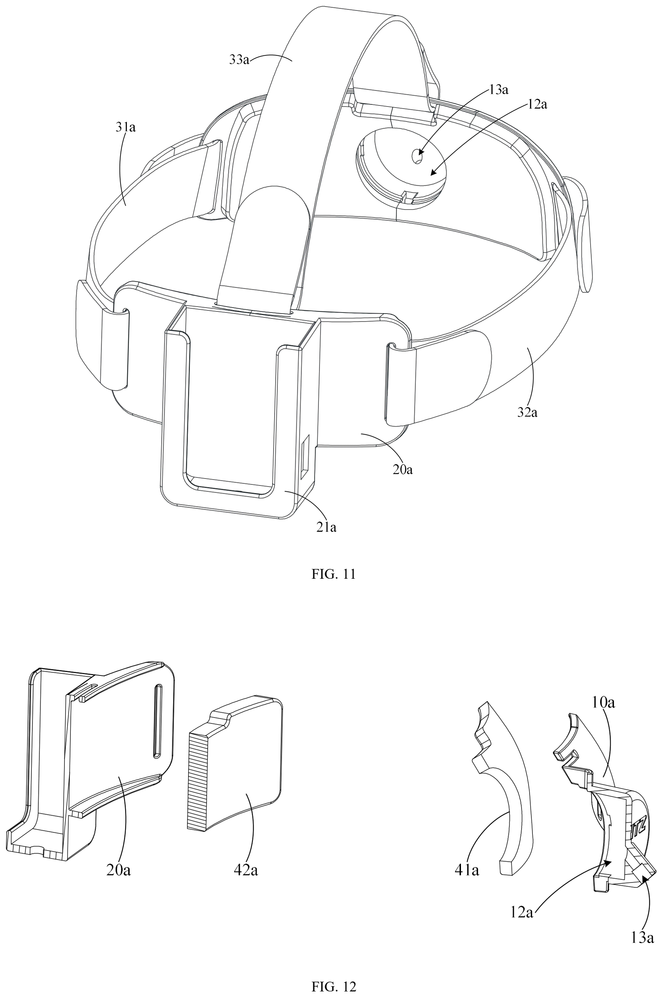

In the embodiments of the present disclosure, as shown in FIGS. 10 and 11, the portable and comfortable head-mounted bracket includes a front bracket 10a, a rear bracket 20a, a first connecting strap 31a, a second connecting strap 32a, and a third connecting strop 33a. The front bracket 10a is configured to be worn on a forehead of a human body. The rear bracket 20a is configured to be worn on an occiput of a human body. The first connecting strap 31a connects a left end of the front bracket 10a to a left end of the rear bracket 20a. The second connecting strap 32a connects a right end of the front bracket 10a to a right end of the rear bracket 20a. The third connecting strap 33a connects a top side edge of the front bracket 10a to a top side edge of the rear bracket 20a.

In this embodiment, the front bracket 10a is configured to support and abut the wearer's forehead, and the rear bracket 20a is configured to support and abut the wearer's occiput. The front bracket 10a and the rear bracket 20a are connected by the first connecting strap 31a, the second connecting strap 32a, and the third connecting strap 33a. A first connection hole is defined in the left end of the front bracket 10a, and a second connection hole is defined in the left end of the rear bracket 20a. A front end of the first connecting strap 31a is connected to the first connection hole, and a rear end of the first connecting strap 31a is connected to the second connection hole, achieving the connection between the first connecting strap 31a and the front bracket 10a and the rear bracket 20a.Connection methods of the second connecting strap 32a and the third connecting strap 33a to the front bracket 10a and the rear bracket 20a may refer to that of the first connecting strap 31a. The first, second, and third connecting straps 31a, 32a, 33a are flexible straps, and lengths thereof are adjustable. A width of the third connecting strap 33a may be set in a range from 25 mm to 30 mm. Such configuration can reduce pressure exerted by the third connecting strap 33a on the wearer's head while reasonably controlling a contact area between the third connecting strap 33a and the wearer.

A front side wall of the front bracket 10a can be configured to be mounted with the imaging device, such that the wearer can wear the imaging device on the forehead for surgical field imaging. By connecting the front bracket 10a and the rear bracket 20a through the first connecting strap 31a, the second connecting strap 32a, and the third connecting strap 33a, the structure between the front bracket 10a and the rear bracket 20a can be simplified, the structural weight between the front bracket 10a and the rear bracket 20a can be reduce, which achieves stable wearing of the head-mounted bracket on the wearer's head, and reduces the weight of the head-mounted bracket, thereby improving the comfort of wearing the head-mounted bracket.

A curvature radius of a rear side of the front bracket 10a ranges from 80 mm to 120 mm; and/or, a curvature radius of the front side of the rear bracket 20a ranges from 80 mm to 120 mm. The curvature radius of the forehead and the occiput of an adult are typically 80 mm to 120 mm. Therefore, setting the curvature radius of the front bracket 10a and rear bracket 20awithin such range can improve the conformity between the front bracket 10a and the wearer's forehead and the conformity between the rear bracket 20a and the wearer's occiput, thereby improving wearing stability and comfort.

In practical applications, a curvature of the rear side of the front bracket 10a is set to 120°to 160°; and/or, a curvature of the front side of the rear bracket 20a is set to 120°to 160°.In this way, the curvature of the front bracket 10a and the rear bracket 20a can conform to the wearer's forehead and occiput, increasing the contact area between the front bracket 10a and the rear bracket 20a and the wearer's head, thereby further improving the wearing stability and comfort of the head-mounted bracket.

For example, as shown in FIG. 12, the head-mounted bracket further includes a front flexible pad 41a mounted on the rear side of the front bracket 10a. The front flexible pad 41a can be a sponge pad or a silicone pad. Compared to the front bracket 10a, the front flexible pad 41a is softer and can directly contact the wearer's skin instead of the front bracket 10a, thereby improving the wearer's skin feel. In addition, the front flexible pad 41a can be deformed to conform to the wearer's forehead better, thereby further enhancing the wearing stability of the head-mounted bracket.

As shown in FIG. 12, the head-mounted bracket further includes a rear flexible pad 42a mounted on a front side of the rear bracket 20a. The rear flexible pad 42a can be a sponge pad or a silicone pad. Compared to the rear bracket 20a, the rear flexible pad 42a is softer and can directly contact the wearer's skin instead of the rear bracket 20a, thereby improving the wearer's skin feel. In addition, the rear flexible pad 42a can be deformed to conform to the back of the wearer's head better, thereby further enhancing the wearing stability of the head-mounted bracket.

For example, as shown in FIG. 11, a mounting bracket 11a is arranged on a rear side wall of the rear bracket 20a. The mounting bracket 11a is configured to be mounted with a control host or a mobile power bank. The control host or the mobile power bank is configured to be electrically connected to the imaging device mounted on the front bracket 10a for providing power or transmitting control signals to the imaging device. The control host or the mobile power bank can be worn on the head-mounted bracket by being mounted on the rear bracket 20a, thereby improving the effectiveness of the head-mounted bracket. In addition, the weight-bearing capacity on the wearer's forehead and back of the head can be more balanced, reducing the burden on the wearer's neck.

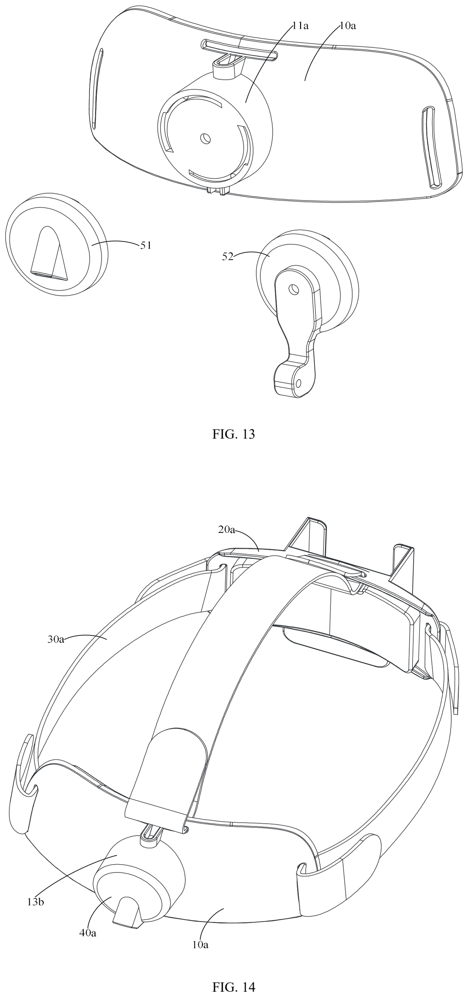

As shown in FIG. 13, a connecting protrusion 11a is arranged on the front wall of the front bracket 10a. The head-mounted bracket further includes a first mounting seat 51 and a second mounting seat 52, which are switchably mounted on the connecting protrusion 11a. The first mounting seat 51 and the second mounting seat 52 are respectively configured to be adaptively mounted with different functional devices mounted thereon. The first mounting seat 51 and the second mounting seat 52 are detachably connected to the connecting protrusion 11a. The first mounting seat 51 can be configured to be adaptively mounted with the imaging device, while the second mounting seat 52 can be configured to be adaptively mounted with a lighting device. In this way, the wearer can replace different mounting seats according to actual needs to switch and install different functional devices, thereby further improving the use effect of the head-mounted bracket.

In practical applications, as shown in FIGS. 11 and 12, the rear wall of the front bracket 10a is recessed to form a wiring groove 12a, and a via 13a is defined in the front wall of the front bracket 10a. The via 13a is in communication with the wiring groove 12a.Taking the installation of the imaging device on the front side wall of the front bracket 10a as an example, the electrical connection wire of the imaging device can extend into the wiring groove 12a through the via 13a. In this way, the wiring groove 12a can receive and hide the electrical connection wire, thereby preventing the electrical connection wire from being exposed and affecting the shooting field of view of the imaging device, and from being directly sandwiched between the front bracket 10a and the user's skin, ensuring the user's wearing comfort.

The present disclosure further provides a surgical field imaging apparatus, which includes an imaging device and a head-mounted bracket. The specific structure of the head-mounted bracket refers to the above embodiments. By adopting all the technical solutions of the above embodiments, the surgical field imaging apparatus at least has all the beneficial effects brought by the technical solutions of the above embodiments, which will not be repeated here. In this embodiment, the imaging device is mounted on a front bracket 10a of the head-mounted bracket.

For example, the surgical field imaging apparatus further includes a control host mounted on a rear side wall of a rear bracket 20a of the head-mounted bracket, and is electrically connected to the imaging device. Users can control the imaging device to capture images through the control host. The surgical field imaging apparatus further includes a gimbal, and the imaging device is mounted on the gimbal. The control host can also control the rotation of the gimbal to adjust the shooting angle of the imaging device. Based on the above embodiment of the mounting bracket 21, the control host can be mounted on the mounting bracket 21, such that the relative position between the control host and the imaging device remains unchanged.

As shown in FIGS. 14 to 17, the present disclosure provides a head-mounted bracket aimed at solving the technical problem of how to improve the convenience of using the head-mounted bracket.

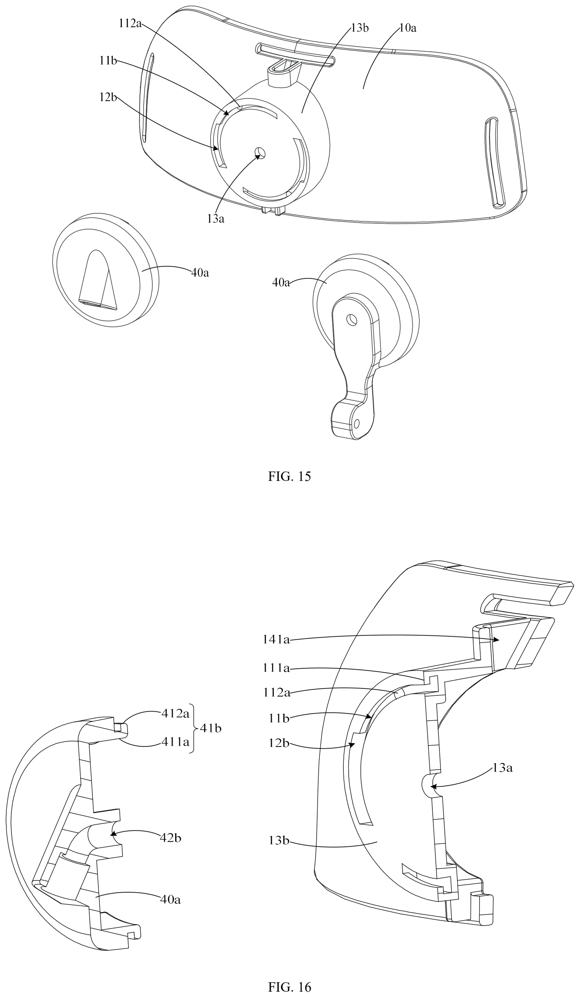

In the embodiments of the present disclosure, as shown in FIGS. 14 and 15, the head-mounted bracket includes a front bracket 10a, a rear bracket 20a, a connecting strap 30a, and at least two mounting seats 40a. The front bracket 10a is configured to be worn on a forehead of a human body, the rear bracket 20a is configured to be worn on an occiput of the human body, and the front bracket 10a is connected to the rear bracket 20a through the connecting strap 30a. Each of the at least two mounting seats 40a is switchably mounted on a front side wall of the front bracket 10a and is adaptively mounted with different functional devices.

In this embodiment, the front bracket 10a is configured to support and abut the wearer's forehead, and the rear bracket 20a is configured to support and abut the wearer's occiput. The front bracket 10a and the rear bracket 20a are connected by three connecting straps 30a, which include a first connecting strap, a second connecting strap, and a third connecting strap. The first connecting strap connects a left end of the front bracket 10a to a left end of the rear bracket 20a, the second connecting strap connects a right end of the front bracket 10a to a right end of the rear bracket 20a, and the third connecting strap connects a top side edge of the front bracket 10a to a top side edge of the rear bracket 20a.The first connecting strap, the second connecting strap, and the third connecting strap are flexible connecting straps, and lengths of the first connecting strap, the second connecting strap, and the third connecting strap are adjustable. A width of the third connecting strap can be set to 25 mm to 30 mm, which can reduce the pressure on the wearer's head caused by the third connecting strap and control the contact area between the third connecting strap and the wearer reasonably.

The number of the mounting seats 40a can be three or more. Each mounting seat 40a can be compatible with the front side wall of the front bracket 10a and is adaptively mounted with different functional devices, such as an imaging device, a lighting device, or magnifying glasses.

Multiple mounting seats 40a can be selectively mounted on the front bracket 10a, and different mounting seats 40a can accommodate different functional devices. By replacing the mounting seats 40a, the functional devices mounted on the head-mounted bracket can be replaced. Users can choose the required functional device to mount on the corresponding mounting seat 40a according to actual needs, and then mount the mounting seat 40a on the front bracket 10a, such that the head-mounted bracket can be compatible with different functional devices, thereby facilitating using the head-mounted bracket.

The mounting seat 40a can be detachably connected to the front bracket 10a through a clamping connection or an insertion connection, which is not limited herein.

As shown in FIGS. 15 to 17, a fixing groove11b is formed in the front bracket 10a,a stopping rib 111a protrudes from a groove wall of the fixing groovellb, and a connecting buckle 41b protrudes from the mounting seat 40a. The connecting buckle 41b detachably engage with the fixing groovellb. The connecting buckle 41b includes a connecting portion 411a and a fastening portion 412a. The connecting portion 411a connects the mounting seat 40ato the fastening portion 412a, the fastening portion 412a is located between the stopping rib 111a and a groove bottom of the fixing groovellb, and the fastening portion 412a is limited and abutted by the stopping rib 111a.

The connecting buckle 41b is in the shape of a "7", with the connecting portion 411a connected to the mounting seat 40a and the buckle portion 412a connected to a side of the connecting portion 411a away from the mounting seat 40a.The stopping rib 111a is arranged at an opening of the fixing groovellb, protruding from one of the groove walls of the fixing groovellb. A fastening space is formed between the stopping rib 111a and the groove bottom of the fixing groovellb. When the connecting buckle 41b engages with the fixing groove11b, the connecting buckle 41b is located in the fastening space and is clamped to the stopping rib 111a. The stopping rib 111a can limit the connecting buckle 41b from leaving the fixing groove11b along a depth direction of the fixing groove11b, which improves the stability of the connection between the mounting seat 40a and the front bracket 10a.

In practical applications, as shown in FIGS. 15 and 16, a sliding groove 12b in communication with the fixing groove 11b is formed in the front bracket 10a. A width of the sliding groove 12b is adapted to a width of the fastening portion 412a, and the fastening portion 412a slidably engages with the sliding groove 12b, such that the fastening portion 412a can slide along the sliding groove 12b into the fixing groovellb or retract from the fixing groovellb to the sliding groove 12b.

When the mounting seat 40ais assembled onto the front bracket 10a, the connecting buckle 41b is placed into the groove 12b at first, and then the connecting buckle 41b is driven to slide along the sliding groove 12b towards the direction of the fixing groove11b, such that the fastening portion 412a enters the fixing groove11b. The fastening portion 412a entering the fixing groove11bis limited and abutted by the stopping rib 111a, thus achieving the installation engagement between the connecting buckle 41b and the fixing groove11b.When the mounting seat 40a is disassembled from the front bracket 10a, the connecting buckle 41b can be driven to slide along the fixing groovellbtowards the direction of the sliding groove 12b, such that the fastening portion 412a exits the fixing groove11b and enters the sliding groove 12b; in this way, the connecting buckle 41b can be detached from the sliding groove 12b, thereby achieving the disassembly of the mounting seat 40a.By sliding the connecting buckle 41b, the disassembly and assembly of the mounting seat 40a and the front bracket 10a can be achieved, which simplifies and facilitates the disassembly and assembly process.

For example, as shown in FIGS. 15 to 17, amounting protrusionl3b protrudes from the front side wall of the front bracket 10a, and the fixing groove 11b and the sliding groove 12b are formed in a front end face of the mounting protrusionl3b. The mounting protrusion 13b can improve the structural strength of the joint between the front bracket 10a and the mounting seat 40a, prevent the front bracket 10a from being easily deformed or damaged after bearing the mechanical stress generated during the disassembly and assembly process of the mounting seat 40a, and extend the overall working life of the head-mounted bracket.

The mounting protrusionl3b can be a circular protrusion, a square protrusion, or other shaped protrusions.

As shown in FIG. 15, the mounting protrusion13b is configured to be circular, and the sliding groove 12b and the fixing groove 11brespectively extend in an arc shape. The mounting seat 40acorresponds to the mounting protrusion 13b in shape and radial dimension, and the connecting buckle 41b is adapted to the shape of the sliding groove 12band the fixing groove 11b.

The sliding groove 12b and the fixing groovellb extend along a circumference of the front end face of the mounting protrusionl3b. During the process of sliding the connecting buckle 41b from the sliding groove 12b into the fixing groovellb, or retracting from the fixing groove11b to the sliding groove 12b, the mounting seat 40a rotates instead of moving in a straight line. This can reduce the space occupied by the movement of the mounting seat 40a during disassembly and assembly, simplify the disassembly and assembly trajectory of the mounting seat 40a, and further facilitate disassembly and assembly of the mounting seat 40a.

In practical applications, two sets of sliding groove 12b and fixing groove11bare configured. The two sets of sliding grooves 12b and fixing grooves 11b are spaced apart along the circumference of the mounting protrusionl3b. The connecting buckles 41b correspond to the sliding groove 12b and the fixing groove 11b in number and position. The mounting seat 40a and the mounting protrusion13b are connected through two fixing grooves 11b and two sets of connecting buckles 41b, which can increase the connection position between the mounting seat 40a and the mounting protrusionl3b, further improving the connection stability.

For example, as shown in FIGS. 15 and 16, a protruding block 112a is arranged on the groove bottom of the fixing groovellb. The protruding block 112a presses and fixes the fastening portion 412a to the stopping rib 111a to increase the resistance when the connecting buckle 41b exits the fixing groove11b. During the process of entering the fixing groove11b, when the fastening portion 412a passes through the protruding block 112a, the fastening portion 412a needs to overcome the resistance caused by the protruding block 12a. After the fastening portion 412a is engageable with the fixing groove11b in place, the protruding block 112a exerts a squeezing force on the fastening portion 412a, such that the fastening portion 412a is more tightly clamped between the protruding block 112a and the stopping rib 111a, which prevents the fastening portion 412a from easily exiting the fixing groove11b. Thus, even if the head-mounted bracket is subjected to impact forces caused by falling, the mounting seat 40a does not easily detach from the front bracket 10a, thus providing protection for the functional device mounted on the mounting seat 40a.

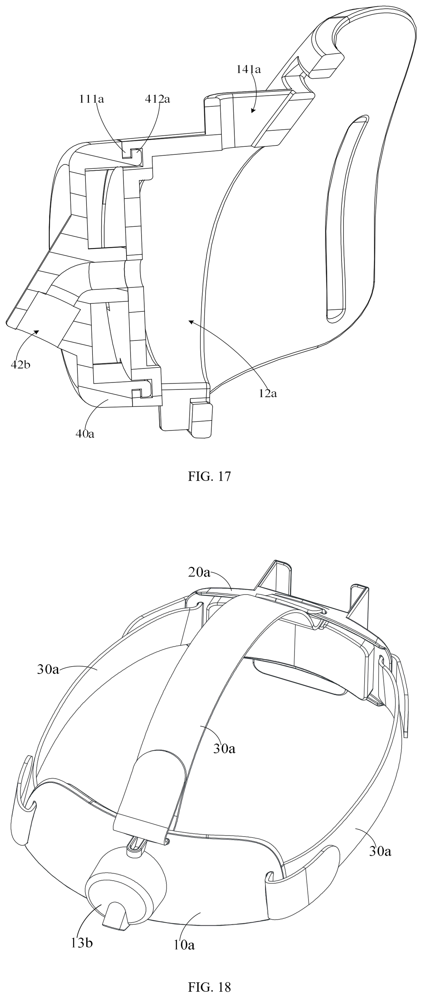

As shown in FIGS. 16 and 17, a via 13a is defined in a center of the mounting protrusionl3b. The via 13a runs through the rear wall of the front bracket 10a. A lead hole 42b corresponding to the via 13a is defined in at least one of the mounting seats 40a.After the functional device is mounted on the mounting seat 40a, the electrical connection wire of the functional device can be sequentially extended to the rear side of the front bracket 10a through the lead hole 42b and the via 13a, thereby preventing the electrical connection wire from being exposed on the front side of the front bracket 10a and affecting user operations.

In practical applications, as shown in FIGS. 16 and 17, an area of the rear wall of the front bracket 10a corresponding to the mounting protrusion13b is recessed to form a wiring groove 12a. A wire passing hole 141a is defined in a highest portion of a groove wall of the wiring groove 12a. The wiring groove 12a can accommodate and hide the electrical connection wire of the functional device, preventing the electrical connection wires from being directly sandwiched between the front bracket 10a and the user's skin and further ensuring the user's wearing comfort.

The present disclosure further provides a surgical field imaging apparatus, which includes an imaging device and a head-mounted bracket. The specific structure of the head-mounted bracket refers to the above embodiments. By adopting all the technical solutions of the above embodiments, the surgical field imaging apparatus at least has all the beneficial effects brought by the technical solutions of the above embodiments, which will not be repeated here. The imaging device is mounted on one of the mounting seats 40a of the head-mounted bracket.

As shown in FIGS. 18 to 20, the present disclosure provides a head-mounted bracket aimed at solving the technical problem of how to improve the use effect of the head-mounted bracket.

In the embodiments of the present disclosure, as shown in FIGS. 18 to 20, the head-mounted bracket includes a front bracket 10a and a rear bracket 20a. The front bracket 10a is configured to be worn on a forehead of a human body. A via 13a is defined in a front side wall of the front bracket 10a, a rear side wall of the front bracket 10a is recessed to form a wiring groove 12a, and the via 13arunning through the wiring groove 12a. The front bracket 10a is configured to be mounted with a functional device, and electrical connection wires of the functional device can extend into the wiring groove 12a through the via 13a. The rear bracket 20ais configured to be worn on an occiput of the human body. The front bracket 10a and the rear bracket 20a are connected through a connecting strap 30a.

In this embodiment, the head-mounted bracket can be worn by the user on the head, with the front bracket 10a supporting and abutting the wearer's forehead, and the rear bracket 20a supporting and abutting the wearer's occiput. The front side wall of the front bracket 10a can be configured to be mounted with the functional device, such that the wearer can wear the functional device on the forehead to assist with work. The functional device needs to be electrically connected to a mobile power bank or a control host through electrical connection wires. After the functional device is mounted on the front bracket 10a, the electrical connection wire can enter the wiring groove 12a through the via 13a, that is, the electrical connection wire can extend to the rear side of the front bracket 10a, and then extend from the rear side of the front bracket 10a to be electrically connected to the mobile power bank or the control host. The redundant part of the electrical connection wire can still be received in the wiring groove 12a.The wiring groove 12a can accommodate and hide the electrical connection wire, thereby preventing the electrical connection wire from being exposed and affecting the user's field of view and the imaging device's imaging field of view, and preventing the electrical connection wire from being directly sandwiched between the front bracket 10a and the user's skin, ensuring the user's wearing comfort.

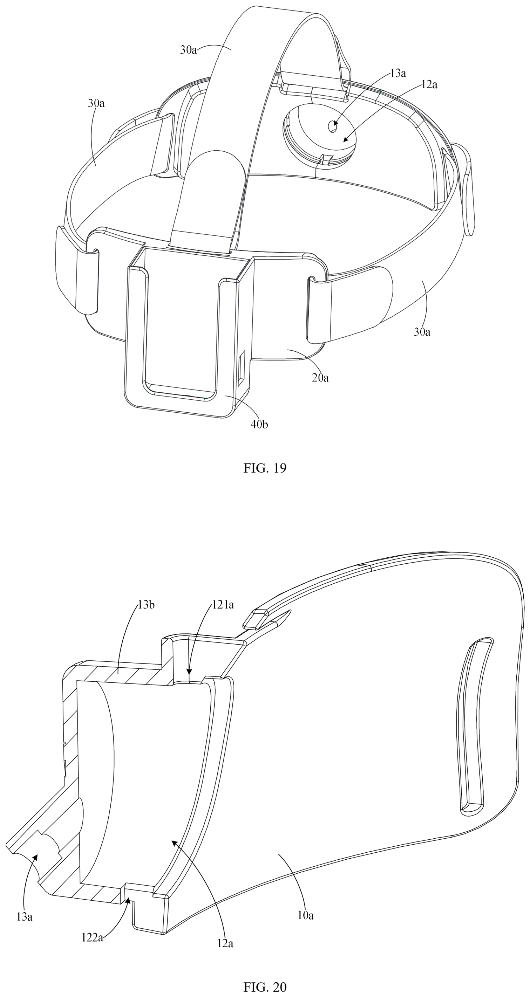

For example, as shown in FIGS. 18 and 19, a mounting protrusion13b protrudes from the front side wall of the front bracket 10a. The mounting protrusion 13b is configured to be mounted with the functional device. The via 13a is defined in the mounting protrusionl3b, and the wiring groove 12a is formed in the area on the rear side wall of the rear bracket 10corresponding to the mounting protrusionl3b.

The mounting protrusion 13b can improve the structural strength of the part of the front bracket 10a where the functional device is mounted, thereby preventing the front bracket 10a from being easily damaged or deformed. The mounting protrusionl3bhas a hollow structure, and a hollow portion serves as the wiring groove 12a, which can increase a depth of the wiring groove 12aand thus increase a capacity of the wiring groove 12a,thereby improving the receiving effect of the electrical connection wire.

As shown in FIGS. 19 and 20, the wiring groove 12a has a groove bottom corresponding to a front end wall of the mounting protrusionl3b, and a groove wall corresponding to a peripheral wall of the mounting protrusionl3b. The via 13a runs through the groove bottom of the wiring groove 12a. The front end wall of the mounting protrusionl3b is configured to be mounted with the functional device. After the functional device is mounted on the mounting protrusionl3b, the electrical connection wire of the functional device can enter the wiring groove 12a from the groove bottom of the wiring groove 12a through the via 13a. This can reduce the length of the electrical connection wire exposed to the via 13a, such that the electrical connection wire can be more and more fully received in the wiring groove 12a.

For example, as shown in FIG. 20, a wire passing hole is defined in the groove wall of the wiring groove 12a, which runs through the peripheral wall of the mounting protrusionl3b.After entering the wiring groove 12a through the via 13a, the electrical connection wire can extend out of the wiring groove 12a through the wire passing hole to connect with the mobile power bank or the control host carried by the user or worn on the head-mounted bracket.

The number of the wire passing hole can be one, two or more, which is not limited here.

As shown in FIG. 20, there are at least two wire passing holes, including a first wire passing hole 121a and a second wire passing hole 122a. The first wire passing hole 121a is located at the highest portion of the groove wall of the wiring groove 12a, and the second wire passing hole 122a is located at the lowest portion of the groove wall of the wiring groove 12a. The first and wire passing holes are arranged opposite each other in the vertical direction. The electrical connection wire can extend out of the wiring groove 12a through one of the first and second wire passing holes, such that the electrical connection wire can be connected to the mobile power bank or the control host. For example, if the user carries a mobile power bank or a control host on their body, the electrical connection wire can extend out of the wiring groove 12a through the second wire passing hole 122a, such that the electrical connection wire can extend downwards to be connected to the mobile power bank or the control host on the user's body. If the user wears the mobile power bank or the control host on the rear bracket 20a, the electrical connection wire can extend out through the first wire passing hole 121a, which allows the electrical connection wires to route around the back of the user's head to be connected to the mobile power bank or the control host. In this way, the first wire passing hole 121a and the second wire passing hole 122a can meet the different wiring needs of users, thereby improving the convenience of using the head-mounted bracket.

For example, as shown in FIG. 19, a mounting bracket 40b is arranged on the rear side wall of the rear bracket 20a. The mounting bracket 40b is configured to be mounted with the control host or the mobile power bank, which allows the control host or the mobile power bank to be worn together on the head-mounted bracket, thereby improving the use effect of the head-mounted bracket. In addition, mounting the control host or the mobile power bank on the rear bracket 20a can allow the weight-bearing capacity on the wearer's forehead and back of the head to be more balanced, reducing the burden on the wearer's neck. After passing through the wire passing hole, the electrical connection wire can extend backwards to be connected to the control host or the mobile power bank, facilitating the electrical connection between the electrical connection wire and the control host or the mobile power bank.

A wiring fixing structure is arranged on the connecting strap 30a to constrain the electrical connection wire of the functional device. The electrical connecting wire can extend along the connecting strap 30a to the rear bracket 20a, and the wire fixing structure constrains the electrical connecting wire on the connecting strap 30a to limit and protect the electrical connection wire, keeping the electrical connection wire close to the connecting strap 30a and avoiding easy hook of the electrical connection wire.

In practical applications, as shown in FIG. 18, multiple connecting straps 30a can be configured, such as a first connecting strap, a second connecting strap, and a third connecting strap. The first connecting strap connects a left end of the front bracket 10a to a left end of the rear bracket 20a, the second connecting strap connects a right end of the front bracket 10a to a right end of the rear bracket 20a, and the third connecting strap connects a top side edge of the front bracket 10a to a top side edge of the rear bracket 20a. The wire fixing structure is located on the third connecting strap.

When the head-mounted bracket is worn on the user's head, the third connecting strap is in contact with the user's head, and the electrical connection wire can extend along the third connecting strap to the rear bracket 20a, that is, extend to the rear bracket 20a through the wear's head. In this way, the electrical connection wire can be kept in a higher position, which further avoids easy hook of the electrical connection wire and improves the power stability of the functional device.

The present disclosure further provides a surgical field imaging apparatus, which includes an imaging device and a head-mounted bracket. The specific structure of the head-mounted bracket refers to the above embodiments. By adopting all the technical solutions of the above embodiments, the surgical field imaging apparatus at least has all the beneficial effects brought by the technical solutions of the above embodiments, which will not be repeated here. The imaging device is mounted on the front bracket 10a of the head-mounted bracket, and the electrical connection wire of the imaging device extend into the wiring groove 12a of the head-mounted bracket through the via 13a of the head-mounted bracket.

The surgical field imaging apparatus further includes a control host, which is mounted on the rear bracket20a of the head-mounted bracket. The electrical connection wire of the imaging device extends along the connecting strap 30a of the head-mounted bracket to be electrically connected to the control host. Users can control the imaging device to capture images by controlling the control host. The surgical field imaging apparatus further includes a gimbal, and the imaging device is mounted on the gimbal. The control host can also control the rotation of the gimbal to adjust the shooting angle of the imaging device. Based on the above embodiment of the mounting bracket 40b, the control host can be mounted on the mounting bracket 40b, such that the relative position between the control host and the imaging device remains unchanged.

The voltage required for the operation of the imaging apparatus (including the imaging device and the gimbal) is usually greater than 5V (such as 9V, 12V), while when the power supply device (mobile power bank or adapter) is directly connected to the imaging apparatus for power supply, the output voltage defaults to be 5V, which cannot meet the voltage required for the operation of the imaging apparatus. The existing solution is to install a battery in a control box of the imaging apparatus, and the power supply device charges the battery of the control box through an interface, and then the battery supplies power to the imaging apparatus, which increases the cost and weight of the control box.

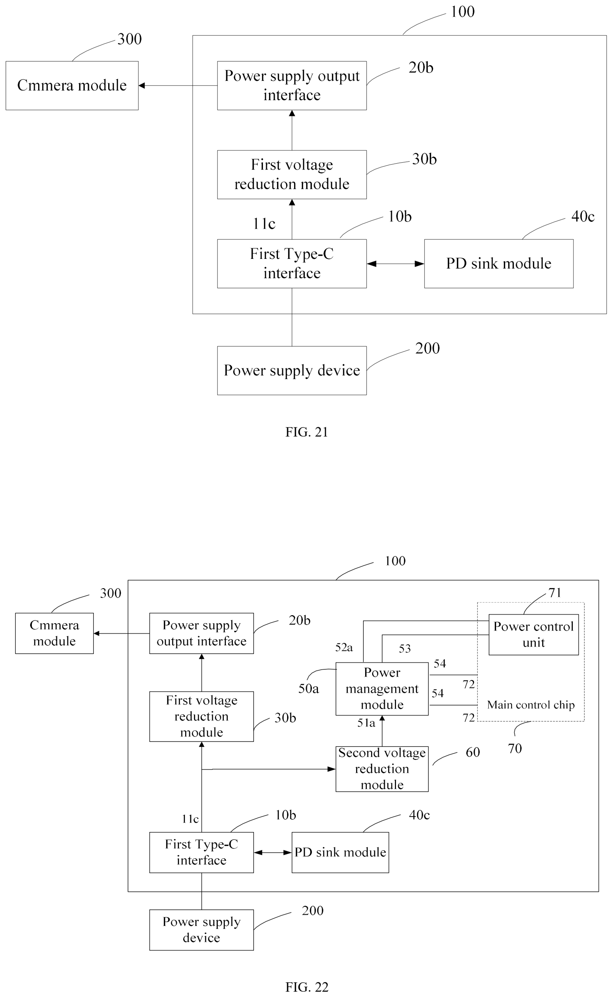

As shown in FIGS. 21 to 24, in response to the aforementioned issues with the camera control box, the present disclosure provides a new camera control box circuit that can effectively reduce the cost and weight of the camera control box. The camera control box circuit of the present disclosure is mainly applied to the camera control box of a camera device.

In this embodiment, the camera control box circuit 100 includes a first Type-C interface 10b, a power supply output interface 20b, a first voltage reduction module 30b, and a power delivery (PD) sink module 40c.

The first Type-C interface 10b is configured to be electrically connected to a power supply device 200 such as a mobile power bank, and a power adapter, etc.

The power supply output interface 20b is configured to be electrically connected to a camera module 300 for supplying power to the camera module 300. The camera module 300 may include a camera lens, a gimbal, and other parts of the camera device.

The first voltage reduction module 30a has an input terminal and an output terminal. The input terminal of the first voltage reduction module 30a is electrically connected to a VBUS pin 11 of the first Type-C interface 10b, and the output terminal of the first voltage reduction module 30a is electrically connected to the power supply output interface 20b.

The PD sink module 40c is electrically connected to the first Type-C interface 10b for protocol communication with the power supply device 200.

In the camera control box circuit 100 of this embodiment, the PD sink module 40c is electrically connected to the first Type-C interface 10b, such that the PD sink module 40c communicates with the power supply device 200 through the first Type-C interface 10b to negotiate a PD protocol, thereby enabling the power supply device 200 to output a voltage exceeding 5V (i.e., the VBUS pin 11 of the first Type-C interface 10b outputs a voltage exceeding 5V), such as 9V, 12V, 15V, 18V, and 20V, according to the PD protocol negotiated with the PD sink module 40c.In this way, the input terminal of the first voltage reduction module 30b can receive the voltage output by the power supply device 200 according to the PD protocol (such as 9V, 12V, and 15V.) through the VBUS pin 11 of the first Type-C interface 10b, and then reduce the voltage received by the VBUS pin 11 of the first Type-C interface 10b to the required operating voltage of the camera module 300 (such as 7.8V), allowing the camera module 300 to work normally.

The camera control box circuit 100 in this embodiment includes the first Type-C interface 10b being electrically connected to the power supply device 200, the power supply output interface 20b for being electrically connected to the camera module 300, the first voltage reduction module 30a, and the PD sink module 40c. The input terminal of the first voltage reduction module 30a is electrically connected to the VBUS pin 11 of the first Type-C interface 10b, and the output terminal of the first voltage reduction module 30a is electrically connected to the power supply output interface 20b. The PD sink module 40c is electrically connected to the first Type-C interface 10b. The PD sink module 40 can communicate with the power supply device 200 through the first Type-C interface 10b, allowing the power supply device 200 to output a voltage exceeding 5V (such as 9V, 12V, and 15V) through the negotiated PD protocol, and the input voltage received by the input terminal of the first voltage reduction module 30bto exceed 5V. The first voltage reduction module 30b then reduce this voltage to the operating voltage required by the camera module 300 (e.g., 7.8V) and supplies the required voltage directly through the power supply output interface 20b, ensuring normal operation of the camera module 300.Compared with the existing technology that requires adding batteries to the camera control box circuit, the technical solution of this embodiment eliminates the need for a battery, thereby reducing the cost, weight, and physical dimensions of the camera control box, significantly enhancing portability of the camera control box, and facilitating the installation of the camera control box.

The first voltage reduction module 30b can use a voltage reduction switch stabilizer to maintain the stable output voltage from the output terminal of the first voltage reduction module, thereby ensuring the stable operation of the camera module 300, for example, the voltage reduction switch stabilizer can be a MP9943GQ chip or other chips with the same function can be used.

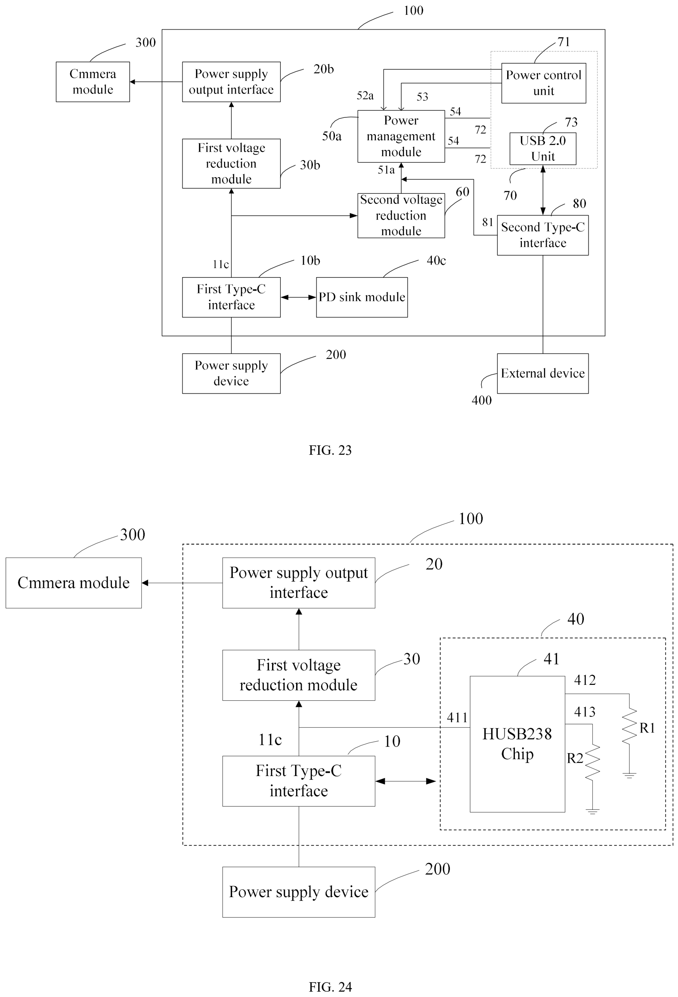

Referring to FIGS. 22 and 23, in some embodiments, the camera control box circuit 100 further includes a power management module 50a, a main control chip 70, and a second voltage reduction module 60.

The power management module 50a includes a voltage input terminal 51a, an enable terminal 52a, a normally-on first voltage output terminal 53, and multiple second voltage output terminals 54.The first voltage output terminal 53 outputs a voltage output whenever the input terminal of the power management module 50a receives a voltage, operating independently of the control state of the enable terminal 52a.

The main control chip 70 includes a power control unit 71 and multiple power supply pins 72. The first voltage output terminal 53 is electrically connected to the power control unit 71 to supply power to the power control unit 71. The multiple power supply pins 72 are respectively electrically connected to the multiple second voltage output terminals 54, and the power control unit 71 is electrically connected to the enable terminal 52a of the power management module 50a, thereby controlling the on and off of the multiple second voltage output terminals 54.In some embodiments, the multiple second voltage output terminals 54 can output voltages of different sizes, such as 0.9V, 1.5V, 1.8V, and 3.0V, respectively, to different power supply pins 72 of the main control chip 70 as different power sources (including a core power supply, a memory power supply, an IO port power supply, etc.).

The input terminal of the second voltage reduction module 60 is electrically connected to the VBUS pin 11 of the first Type-C interface 10b, and the output terminal of the second voltage reduction module 60 is electrically connected to the voltage input terminal 51a of the power management module 50a.

In this embodiment, the second voltage reduction module 60 receives the voltage output by the power supply device 200 according to the PD protocol (such as 9V and 12V,) from the VBUS pin 11 of the first Type-C interface 10b, reduces the received voltage to a specified voltage (such as 4.8V), and outputs the specified voltage to the input terminal of the power management module 50a. After receiving the voltage output by the second voltage reduction module 60, the power management module 50a outputs a preset voltage (e.g. 1.8V) to the power control unit 71 of the main control chip 70 through the first voltage output terminal 53 thereof. The power control unit 71 then starts working and controls the output of the corresponding signal to the enable terminal 52a of the power management module 50a, allowing the power management module 50a to turn on each second voltage output terminal 54 to supply power to each power supply pin 72 of the main control chip 70. Therefore, each power supply pin 72 of the main control chip 70 supplies power to the corresponding device part, enabling the camera control box to start working normally.

The power control unit 71 of the main control chip 70 can control the electrical signal of the enable terminal 52a of the power management module 50a, thereby controlling the power management chip to turn off each second voltage output terminal 54 and disconnecting the power supply of each power supply pin 72 of the main control chip 70.

The second voltage reduction module 60 can be a single output voltage reduction converter, such as a SY8623 chip. It is noted that the second voltage reduction module 60 can be other chips with the same function. The power management module 50a can be a I2225 chip or other chips with the same function.

The main control chip 70 can be the SOC of the camera control box circuit 100, for example, a V39M can be selected as the main control chip 70.

The technical solution of this embodiment, although the voltage of the VBUS pin 11 of the first Type-C interface 10b is increased to exceed 5V through the PD sink module 40c, the voltage of the VBUS pin 11 still can be reduced to the level required by the main control chip 70 and the power management module 50a through the second voltage reduction module 60, without affecting the normal operation of the main control chip 70 and the power management module 50a.

Referring to FIG. 23, in some embodiments, the camera control box circuit 100 further includes a second Type-C interface 80 configured to connect an external device 400 (such as a burning device and a storage device).The main control chip 70 further includes a USB 2.0 unit 73, and a VBUS pin 81 of the second Type-C interface 80 is electrically connected to the voltage input terminal 51a of the power management module 50a. A D+ pin and a D- pin of the second Type-C interface 80 are electrically connected to the USB 2.0 unit 73 for data transmission.

In this embodiment, the external device such as the storage device or the burning device 400 can be connected to the second Type-C interface 80 to transfer data with the USB 2.0 unit 73 of the main control chip 70, such that the burning device can burn programs to the main control chip 70, or the storage device can transfer image data, video data, and other data with the camera control box. When the external device 400 is connected to the second Type-C interface 80, if the first Type-C interface 10b is not connected to the power supply device 200, the external device 400 can supply power to the power management module 50a through the VBUS pin 81 of the second Type-C interface 80, such that the power management module 50a can supply power to the main control chip 70 and related devices through the first voltage output terminal 53 and each second voltage output terminal 54, enabling the camera control box to perform data transmission work normally.

In some embodiments, a diode is connected in series between the VBUS pin 81 of the second Type-C interface 80 and the voltage input terminal 51a of the power management module 50a, and an anode of the diode is electrically connected to the VBUS pin 81 of the second Type-C interface 80, that is, the diode conducts unidirectionally from the VBUS pin 81 of the second Type-C interface 80 to the voltage input terminal 51a of the power management module 50a. In this way, when the first Type-C interface 10b is connected to the power supply device 200 and the second Type-C interface 80 is connected to the external device 400, the voltage output by the second voltage reduction module 60 can be prevented from being outputted to the external device 400 through the VBUS pin 81 of the second Type-C interface 80, such that the power supply device 200 can maintain a longer power supply time.

Referring to FIG. 24, in some embodiments, the PD sink module 40c includes a HUSB238 chip 41c. A XIN pin 411b of the HUSB238 chip 41c is electrically connected to the VBUS pin 11 of the first Type-C interface 10b. A D+ pin, a D- pin, a CC1 pin, and a CC2 pin of the HUSB238 chip 41c respectively correspond to a D+ pin, a D- pin, a CC1 pin, and a CC2 pin of the first Type-C interface 10b. The HUSB238 chip is a highly integrated USBPD sink chip with a transmission rated power of up to 100W, which is compatible with a PD3.0V1.3 and a Type-CV1.4, and can also support charging protocols such as BC1.2DCP, CDP, and SDP.

In this embodiment, the HUSB238 chip 41c supplies power through the VBUS pin 11 of the first Type-C interface 10b, and communicates and negotiates protocols with the device connected to the first Type-C interface 10b through the D+, D -, CCl, CC2 pins. By using the HUSB238 chip 41c, the VUBS pin of the first Type-C interface 10b can output various required voltages of different levels, and the required voltage can be set according to operational demands, thereby supplying powers to different camera modules 300 requiring various operating voltages.

In some embodiments, an ISET pin 412b of the HUSB238 chip 41c is grounded through a first resistor R1. The first resistor R1 is configured to set a current magnitude, i.e., to set an output current magnitude of the VBUS pin 11 of the first Type-C interface 10b. For example, the first resistor R1 can be 4.53 kΩ, 7.5 kΩ, 10.5 kΩ, 13.7 k Ω, 16.5 kΩ, 19.6 kΩ, 22.6 kΩ, and the corresponding output currents of the VBUS pin 11 of the first Type-C interface 10b are 1.5A, 1.75A, 2A, 2.25A, 2.5A, 2.75A, and 3A, respectively. In some embodiments, A VSET pin 413a of the HUSB238 chip 41c is grounded through a second resistor R2. The second resistor R2 is configured to set a voltage level, i.e., to set an output voltage level of the VBUS pin 11 of the first Type-C interface 10b.For example, the second resistor R2 can be 6.04 kΩ, 10 k Ω, 14 k Ω, or 17.8 k Ω, and the corresponding output voltages of the VBUS pin 11 of the first Type-C interface 10b are 9V, 12V, 15V, and 18V, respectively. Users can choose the first resistor R1 and the second resistor R2 according to operational power requirements. For example, when the first resistor R1 is selected as 10.5 kΩ and the second resistor R2 is selected as 10 k Ω, the output power of the VUBS pin of the first Type-C interface 10b is 24W.

In some embodiments, the ISET pin 412b of the HUSB238 chip 41c can also be suspended. At this time, the HUSB238 chip 41c recognizes all PD protocols of the power supply device 200, and the VBUS pin 11 of the first Type-C interface 10b outputs a voltage based on the maximum output current deliverable by the power supply device 200 can output.

In some embodiments, the VSET pin 413a of the HUSB238 chip 41c can also be suspended. At this time, the HUSB238 chip 41c recognizes all PD protocols of the power supply device 200, and the VBUS pin 11 of the first Type-C interface 10b outputs a voltage based on the maximum output voltage deliverable by the power supply device 200.

It is noted that in other embodiments, the PD sink module 40c may also include other models of USB PD sink chips.

It should be noted that the above embodiments of this embodiment can be freely combined to form new embodiments without any contradictions or conflicts.

The present disclosure further provides a camera control box including the camera control box circuit described above. The specific structure of the camera control box circuit refers to the above embodiments. By adopting all the technical solutions of the above embodiments of the camera control box circuit, the camera control box has at least all the beneficial effects brought by the technical solutions of the above embodiments, which will not be repeated here.

The present disclosure further provides an imaging apparatus, including the camera module, the power supply device, and the camera control box mentioned above. The specific structure of the camera control box refers to the above embodiments. By adopting all the technical solutions of the above embodiments of the camera control box, the imaging apparatus at least has all the beneficial effects brought by the technical solutions of the above embodiments, which will not be repeated here. The first Type-C interface of the camera is electrically connected to the power supply device, and the power supply output interface is electrically connected to the camera module to supply power to the camera module.

The above descriptions are merely optional embodiments of the present disclosure and do not thereby limit the scope of the invention. Any equivalent structural transformations made under the inventive concept of the present disclosure, utilizing the contents of the specification and accompanying drawings, or directly/indirectly applied in other related technical fields, are all included within the scope of patent protection of the present disclosure.

## Claims

1. A imaging device comprising:
a housing having a mounting chamber and a camera hole being in communication with the mounting chamber;
a lens module mounted in the mounting chamber, wherein the lens module comprises a lens and an imaging unit, one end of the lens is located at the camera hole to capture optical images through the camera hole, and the other end of the lens is detachably connected to the imaging unit, allowing the lens to be replaceable; and
a protective cover detachably covered on the camera hole and having a transparent area corresponding to the lens.

2. The imaging device as claimed in claim 1, wherein a connection hole is defined in the imaging unit, and one end of the lens detachably engages with the connection hole.

3. The imaging device as claimed in claim 2, wherein an engagement depth between the lens and the connection hole is adjustable to allow for focus adjustment of the lens.

4. The imaging device as claimed in claim 3, wherein a peripheral wall of an engagement portion between the lens and the connection hole is provided with external threads, a hole wall of the connection hole is provided with internal threads, and the lens engages with the connection hole through threaded engagement.

5. The imaging device as claimed in claim 1, wherein the lens extends out of the camera hole, the protective cover comprises a cover plate and a surrounding plate, the surrounding plate is connected to a periphery of the cover plate, the transparent area is formed on the cover plate, the cover plate corresponds to the lens, and the surrounding plate is arranged around a portion of the lens which extends out of the camera hole.

6. The imaging device as claimed in claim 5, wherein the imaging device further comprises an installation ring, the installation ring is arranged inside the camera hole and surrounding the lens, and one end of the surrounding plate away from the cover plate is detachably connected to the installation ring.

7. The imaging device as claimed in claim 6, wherein a buckle protrudes from the end of the surrounding plate away from the cover plate, and a buckle hole adapted to the buckle is defined in the installation ring, and the buckle detachably engages with the buckle hole.

8. An imaging apparatus, comprising an installation gimbal and an imaging device, wherein the installation gimbal is configured to be fixedly mounted with an external device, and the imaging device is rotatably mounted on the installation gimbal;
the imaging device comprises a housing, a lens module, and a protective cover; the housing has a mounting chamber and a camera hole in communication with the mounting chamber; the lens module is mounted in the mounting chamber, comprising a lens and an imaging unit; one end of the lens is located at the camera hole to capture optical images through the camera hole, and the other end of the lens is detachably connected to the imaging unit to allow the lens to be replaceable; and the protective cover is detachably covered on the camera hole, and has a transparent area corresponding to the lens.

9. The imaging apparatus as claimed in claim8, wherein an outer surface of the housing is recessed to form a first mounting groove; a first through hole is defined in the installation gimbal and is in communication with an inner cavity of the installation gimbal, and the first through hole corresponds to an opening of the first mounting groove; the imaging apparatus further comprises a first brushless motor comprising a first fixing seat, a first stator, and a first rotor; the first fixing seat comprises a first fixing plate and a first connecting shaft protruding from the first fixing plate; the first fixing plate is mounted in the inner cavity of the installation gimbal, and the first connecting shaft extends out of the installation gimbal through the first through hole and extends into the first mounting groove; the first stator and the first rotor are received in the first mounting groove, the first stator is fixed to the first connecting shaft, the first rotor is arranged around the first stator and is rotatably connected to the first connecting shaft, and the first rotor is fixed to the first mounting groove to drive the imaging device to rotate.

10. The imaging apparatus as claimed in claim 9, wherein the installation gimbal comprises a fixing gimbal and a movable gimbal, the fixing gimbal is fixedly mounted to an external device, the movable gimbal is L-shaped, the movable gimbal comprises a connecting portion and an installation portion connected at an angle, the connecting portion is rotatably mounted on the fixing gimbal, the imaging device is located on one side of the connecting portion away from the fixing gimbal, the first fixing plate is mounted in the inner cavity of the installation portion, the first through hole is defined in the installation portion, and a rotation axis of the connecting portion is perpendicular to a rotation axis of the imaging device.

11. The imaging apparatus as claimed in claim 10, wherein one side of the connecting portion facing the fixing gimbal is recessed to form a second mounting groove, and a second through hole is defined in the fixing gimbal and is in communication with an inner cavity of the fixing gimbal; the second through hole corresponds to an opening of the second mounting groove; the imaging apparatus further comprises a second brushless motor comprising a second fixing seat, a second stator, and a second rotor; the second fixing seat comprises a second fixing plate and a second connecting shaft protruding from the second fixing plate; the second fixing plate is mounted in the inner cavity of the fixing gimbal, and the second connecting shaft extends out of the fixing gimbal through the second through hole and extends into the second mounting groove; the second stator and the second rotor are received in the second mounting groove, the second stator is fixed to the second connecting shaft, the second rotor is arranged around the second stator and is rotatably connected to the second connecting shaft, and the second rotor is fixedly connected to the second mounting groove to drive the movable gimbal to rotate.

12. The imaging apparatus as claimed in claim 11, wherein the imaging apparatus further comprises a main control board mounted inside the fixing gimbal, a second wiring channel is formed in the second connecting shaft, one end of the second wiring channel runs through the end of the second connecting shaft and the other end of the second wiring channel runs through the second fixing plate; a second wiring hole is defined in a bottom of the second mounting groove and is in communication with an inner cavity of the connecting portion; the first brushless motor further comprises a first electrical connector; one end of the first electrical connector is mounted on the first fixing plate and is electrically connected to the first stator, and the other end of the first electrical connector extends from inside the installation portion along the connecting portion, the second wiring hole, and the second wiring channel in sequence into the fixing gimbal, and is electrically connected to the main control board.

13. The imaging apparatus as claimed in claim 12, wherein a first wiring channel is formed in the first connecting shaft, one end of the first wiring channel runs through an end of the first connecting shaft and the other end of the first wiring channel runs through the first fixing plate; a first wiring hole is defined in a bottom of the first mounting groove and the first wiring hole connects an inner cavity of the housing with the first wiring channel; an electrical connection wire of the lens module extends from the housing along the first wiring hole, the first wiring channel, the installation portion, the connecting portion, the second wiring hole, and the second wiring channel in sequence to the fixing gimbal, and is electrically connected to the main control board.

14. The imaging apparatus as claimed in claim 13, wherein the second brushless motor further comprises a second electrical connector; one end of the second electrical connector is mounted on the second fixing plate and is electrically connected to the second stator, and the other end of the second electrical connector is electrically connected to the main control board.

15. The imaging apparatus as claimed in claim 8,an outer surface of the housing is recessed to form a first mounting groove; a first through hole is defined in the installation gimbal and is in communication with an inner cavity of the installation gimbal; the first via corresponds to an opening of the first mounting groove; the imaging apparatus further comprises a first brushless motor comprising a first fixing seat, a first stator, and a first rotor; the first fixing seat comprises a first fixing plate and a first connecting shaft protruding from the first fixing plate; the first fixing plate is mounted in the inner cavity of the installation gimbal, and the first connecting shaft extends out of the installation gimbal through the first through hole and extends into the first mounting groove; the first stator and the first rotor are received in the first mounting groove, the first stator is fixed to the first connecting shaft, the first rotor is arranged around the first stator and is rotatably connected to the first connecting shaft,, and the first rotor is fixed to the first mounting groove to drive the imaging device to rotate.
